# EUROPEAN PATENT APPLICATION

(11) **EP 4 623 981 A1**
(43) Date of publication of application: **01.10.2025**
(21) Application number: 24750178.6
(22) Date of filing: 29.01.2024
(51) Int. Cl.: A61M 25/06, A61M 5/158

(54) **BLOOD VESSEL PIERCING DEVICE AND BLOOD VESSEL PIERCING SYSTEM**

(30) Priority: 02.02.2023 JP 2023014501
(71) Applicant: Terumo Kabushiki Kaisha, Tokyo 151-0072 (JP)
(72) Inventor: TSUMAKI, Shota, Nakakoma-gun, Yamanashi 409-3853 (JP); YOKOKUME, Takayuki, Nakakoma-gun, Yamanashi 409-3853 (JP)
(74) Representative: KIPA AB
(86) International application number: PCT/JP2024/002541
(87) International publication number: WO 2024/162229

(57) **Abstract**

The present invention relates to a blood vessel puncture device (10) and a blood vessel puncture system (12). The blood vessel puncture device (10) of the blood vessel puncture system (12) includes a needle body (36), a needle hub (38), and a light source unit (20), and a distal end of the needle body includes a blade surface (42) and a distal end opening (44). In the lumen (40) of the needle body, a light guide member (22) for guiding light emitted by the light source unit to the distal end opening is arranged, the light guide member includes a light guide distal end (82) protruding in a distal direction from a proximal end (80) of the distal end opening, and an outer surface of the light guide distal end includes a light emission unit (88) that emits light (L1) in a direction in which the distal end opening faces, and a light non-transmissive portion (78) that prevents emission of light.

## Description

### Technical Field

The present invention relates to a blood vessel puncture device and a blood vessel puncture system.

### Background Art

For example, JP 2018-171231 A discloses a blood vessel puncture device including a needle body capable of puncturing a blood vessel of a living body site, a needle hub provided at a proximal end of the needle body, a light source unit provided in the needle hub, and a light guide member that guides light emitted by the light source unit to a distal end of the needle body. A first lumen through which a guide wire can be inserted and a second lumen in which the light guide member is arranged are formed in the needle body. The distal end of the needle body is provided with a blade surface on which a distal end opening communicating with the first lumen is formed, and an exposure hole communicating with the second lumen to expose a distal end of the light guide member. The exposure hole is located in a proximal direction from the distal end opening.

According to such a blood vessel puncture device, light derived from the light guide member through the exposure hole is absorbed or attenuated by blood, so that before and after the exposure hole formed at the distal end of the needle body is inserted into the blood vessel, intensity of the light that can be received or visually recognized outside the living body site changes. Therefore, it is possible to know that the distal end of the needle body is inserted into the blood vessel on the basis of the light emitted by the light source unit.

### Summary of Invention

However, in the above-described blood vessel puncture device, since the exposure hole is located in a proximal direction from the distal end opening of the needle body, there is a possibility that it is not possible to promptly know that the distal end opening is inserted into the blood vessel.

An object of the present invention is to solve the above problems.
(1) A first aspect of the present invention is a blood vessel puncture device including a tubular needle body capable of puncturing a blood vessel of a living body site, a needle hub provided at a proximal end of the needle body, and a light source unit provided in the needle hub, a distal end of the needle body including a blade surface, and a distal end opening formed on the blade surface and communicating with a lumen of the needle body, in which a light guide member for guiding light emitted by the light source unit to the distal end opening is arranged in the lumen of the needle body, the light guide member includes a light guide distal end protruding in a distal direction from a proximal end of the distal end opening, and an outer surface of the light guide distal end includes a light emission unit that emits the light in a direction in which the distal opening faces, and a light non-transmissive portion that prevents emission of the light.
   According to such a configuration, the light emitted by the light source unit is emitted from the light emission unit of the light guide distal end through the distal end opening. Therefore, it is possible to quickly know that the distal end opening of the needle body is inserted into the blood vessel. Since the outer surface of the light guide distal end includes the light non-transmissive portion, an amount of the light emitted from the light guide distal end can be suppressed. As a result, irregular reflection in skin tissue is suppressed and a light emitting region of the light guide distal end is narrowed, so that a position of the distal end opening of the needle body in the skin tissue can be known more accurately. Therefore, the needle body can accurately puncture the blood vessel.
(2) The blood vessel puncture device according to the item (1) described above, in which the light non-transmissive portion may be provided on a distal end face of the light guide distal end.
   According to such a configuration, since the amount of light emitted in the distal direction of the needle body can be suppressed, the light emitting region of the light guide distal end in the skin tissue can further be narrowed. Therefore, the needle body can more accurately puncture the blood vessel.
(3) The blood vessel puncture device according to the item (2) described above, in which the light non-transmissive portion may be provided on an entire distal end face of the light guide distal end.
   According to such a configuration, it is possible to further suppress the irregular reflection in the skin tissue.
(4) The blood vessel puncture device according to any one of the items (1) to (3) described above, in which the light emission unit and the light non-transmissive portion may be provided on an outer peripheral surface of the light guide distal end.
   According to such a configuration, since unnecessary emission of the light from the outer peripheral surface of the light guide distal end can be suppressed, the irregular reflection in the skin tissue can further be suppressed.
(5) The blood vessel puncture device according to any one of the items (1) to (4) described above, in which the light emission unit may extend in an axial direction of the light guide member.
(6) The blood vessel puncture device according to any one of the items (1) to (4) described above, in which a plurality of light emission units may be arranged at intervals in an axial direction of the light guide member.
   According to such a configuration, when the distal end opening of the needle body is inserted into the blood vessel, the light emission unit becomes invisible stepwise from a distal direction. As a result, the user can easily grasp to what extent the distal end opening of the needle body is inserted into the blood vessel.
(7) The blood vessel puncture device according to any one of the items (1) to (4) described above, in which the light emission unit may extend annularly in a circumferential direction of the light guide distal end.
   According to such a configuration, it is not necessary to position the light emission unit and the distal end opening in the circumferential direction of the light guide distal end.
(8) The blood vessel puncture device according to the item (4) described above, in which a length of the light emission unit in a circumferential direction of the light guide distal end may be equal to or less than a half circumference of the light guide distal end.
   According to such a configuration, it is possible to efficiently suppress the irregular reflection in the skin tissue.
(9) The blood vessel puncture device according to any one of the items (1) to (8) described above, in which the light source unit may emit near infrared light.
   According to such a configuration, since the near-infrared light is more easily transmitted through the skin tissue and absorbed by hemoglobin than visible light, it is possible to more easily know that the distal end opening is inserted into the blood vessel.
(10) The blood vessel puncture device according to any one of the items (1) to (9) described above, in which the light source unit may be detachably attached to the needle hub.
   According to such a configuration, it is possible to select whether or not to cause the light guide distal end to emit light according to a position of the blood vessel to be punctured and a puncturing technique of the user. The light source unit can be reused.
(11) The blood vessel puncture device according to the item (10) described above, in which the light guide member may be provided in the light source unit, and an inner surface of the needle hub may include a guide surface tapered in diameter toward the lumen of the needle body.
   According to such a configuration, when the light source unit is attached to the needle hub, the light guide member can be easily inserted into the lumen of the needle body by the guide surface.
(12) The blood vessel puncture device according to any one of the items (1) to (11) described above, in which the needle body or the light guide member may be provided with a blood introduction path for guiding blood to the needle hub, and the needle hub may be provided with a blood inflow portion for confirming flashback into which the blood guided from the blood introduction path flows.
   According to such a configuration, it is possible to reconfirm that the distal end opening is inserted into the blood vessel by the inflow of the blood into the blood inflow portion.
(13) The blood vessel puncture device according to the item (12) described above, in which the needle hub may be provided with a blood circulation prevention unit that prevents the blood flowing into the blood inflow portion from circulating in the light source unit.
   According to such a configuration, the blood circulation prevention unit can prevent the light source unit from coming into contact with the blood. Therefore, in a case where the light source unit is detachably attached to the needle hub, the light source unit can be reused.
(14) The blood vessel puncture device according to any one of the items (1) to (13) described above, capable of including a tubular catheter shaft including a lumen through which the needle body is inserted, and a catheter hub provided at a proximal end of the catheter shaft and including a lumen through which the needle body is inserted.
(15) A second aspect of the present invention is a blood vessel puncture system including the blood vessel puncture device according to any one of the items (1) to (14) described above, a light reception unit that receives the light derived from the light emission unit, and an image display unit that displays a received light image created on the basis of the light received by the light reception unit.

According to such a configuration, a position of the light guide distal end can be easily confirmed even in a case where the light emitted from the light guide distal end is difficult to be visually recognized or cannot be visually recognized with the naked eye.

According to the present invention, it is possible to quickly know that the distal end opening of the needle body is inserted into the blood vessel.

### Brief Description of Drawings

Fig. 1 is a schematic configuration diagram of a blood vessel puncture system according to an embodiment of the present invention.
Fig. 2 is an exploded perspective view of the blood vessel puncture device.
Fig. 3 is a longitudinal cross-sectional view of the blood vessel puncture device.
Fig. 4A is an enlarged view of a distal end of the blood vessel puncture device. Fig. 4B is a plan view of the blood vessel puncture device of Fig. 4A.
Fig. 5 is a first illustrative diagram of a puncturing procedure to a blood vessel of the blood vessel puncture device.
Fig. 6 is an illustrative diagram illustrating a received light image in a state of Fig. 5.
Fig. 7 is a second illustrative diagram of the puncturing procedure.
Fig. 8 is an illustrative diagram illustrating a received light image in a state of Fig. 7.
Fig. 9 is a third illustrative diagram of the puncturing procedure.
Fig. 10A is a partially abbreviated plan view of a blood vessel puncture device according to a first modification. Fig. 10B is a partially abbreviated plan view of a blood vessel puncture device according to a second modification.
Fig. 11 is a longitudinal cross-sectional view of a blood vessel puncture device according to a configuration example.

### Description of Embodiments

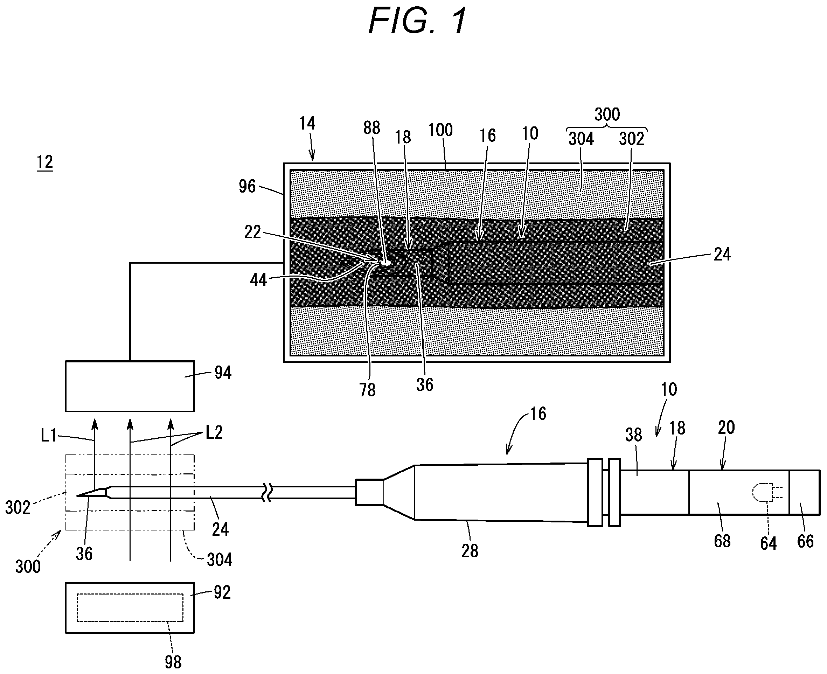

As illustrated in Fig. 1, a blood vessel puncture system 12 according to an embodiment of the present invention is provided with a blood vessel puncture device 10 and a visualization device 14. The blood vessel puncture device 10 is configured as an indwelling needle (peripheral arteriovenous indwelling needle, dialysis indwelling needle and the like) for administering an infusion solution (medical solution) into a blood vessel 302 of a living body site 300. Note that, the blood vessel puncture device 10 may be a blood vessel access product of a peripherally inserted central venous catheter (PICC), a midline catheter, or a central venous catheter. The blood vessel puncture device 10 may be a blood sampling needle without catheter.

As illustrated in Figs. 1 to 3, the blood vessel puncture device 10 includes a catheter member 16, a needle member 18, a light source unit 20, and a light guide member 22. The catheter member 16, the needle member 18, and the light guide member 22 are disposable products that are discarded after one use. The light source unit 20 is a reusable product that can be used a plurality of times.

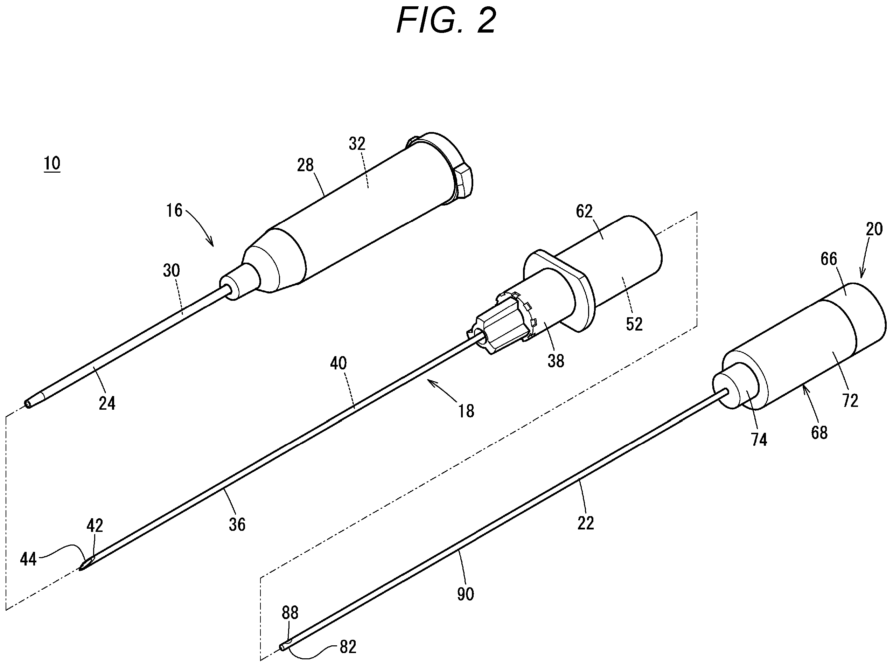

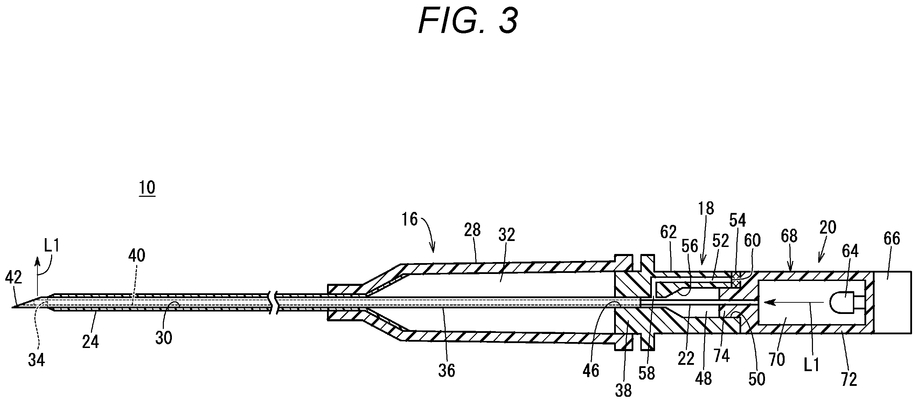

As illustrated in Figs. 2 and 3, the catheter member 16 includes a flexible catheter shaft 24 and a catheter hub 28 provided at a proximal end of the catheter shaft 24. The catheter shaft 24 is a tubular member that can be persistently inserted into the blood vessel 302 of the living body site 300. The catheter shaft 24 includes a lumen 30 (medical solution supply path) extending in an axial direction over an entire length thereof. A distal end of the catheter shaft 24 is opened.

Examples of a constituent material of the catheter shaft 24 include a fluorine-based resin such as polytetrafluoroethylene (PTFE), an ethylene-tetrafluoroethylene copolymer (ETFE), and a perfluoroalkoxy fluorine resin (PFA), an olefin-based resin such as polyethylene and polypropylene or a mixture thereof, polyurethane, polyester, polyamide, a polyether nylon resin, and a mixture of the olefin-based resin and an ethylene-vinyl acetate copolymer.

As illustrated in Fig. 3, the catheter hub 28 is formed in a hollow shape (cylindrical shape). The proximal end of the catheter shaft 24 is fixed to a distal end of the catheter hub 28. The catheter hub 28 is preferably formed of a material harder than that of the catheter shaft 24. A constituent material of the catheter hub 28 is not particularly limited, and a thermoplastic resin such as polypropylene, polycarbonate, polyamide, polysulfone, polyarylate, a methacrylate-butylene-styrene copolymer, polyurethane, an acrylic resin, or an ABS resin can be suitably used, for example. A lumen 32 of the catheter hub 28 may be provided with a hemostasis valve not illustrated.

As illustrated in Figs. 2 and 3, the needle member 18 includes a needle body 36 and a needle hub 38 provided at a proximal end of the needle body 36. The light guide member 22 is inserted into the needle member 18 from a site in a proximal direction from the needle hub 38 to a distal end of the needle body 36 (refer to Fig. 3).

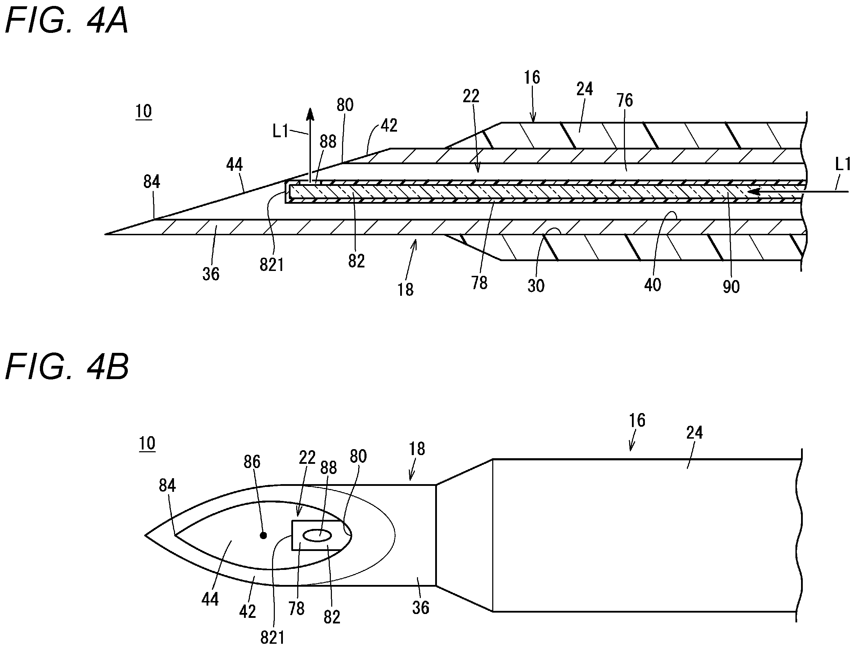

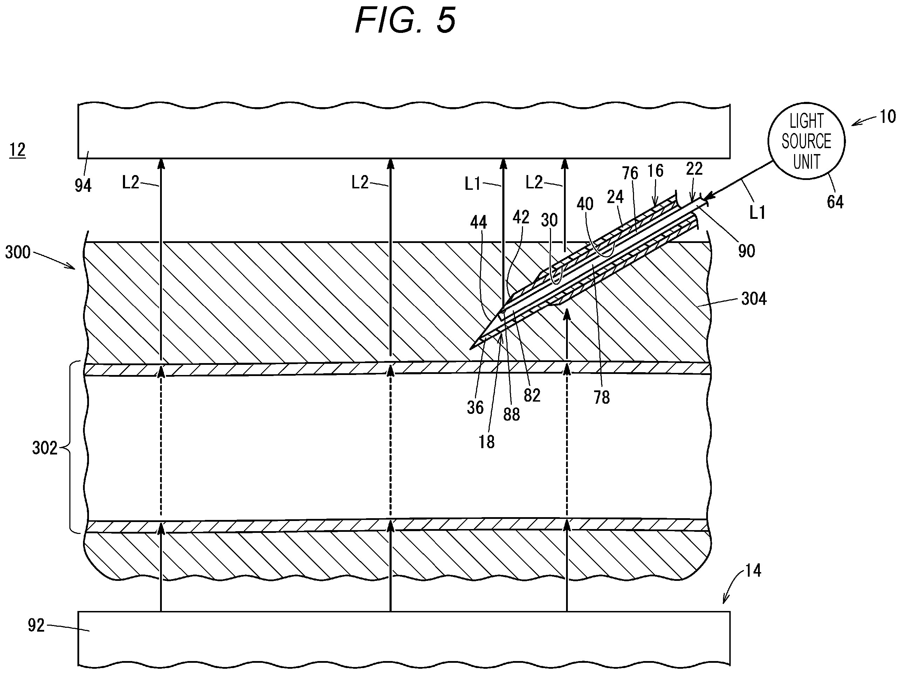

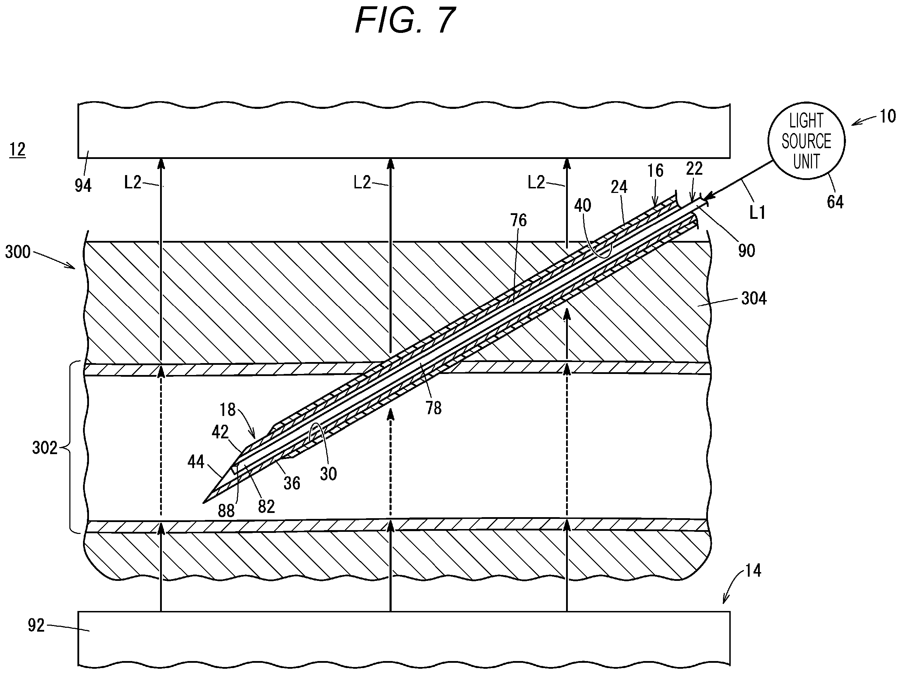

The needle body 36 is a tubular member having rigidity capable of puncturing the living body site 300 (refer to Figs. 5 and 7). The needle body 36 is formed in a circular tube shape. The needle body 36 includes a lumen 40 extending in an axial direction (refer to Figs. 2 and 4A). As illustrated in Fig. 3, the needle body 36 is inserted into the lumen 30 of the catheter shaft 24 and the lumen 32 of the catheter hub 28 in an initial state (assembled state) of the blood vessel puncture device 10. The light guide member 22 is inserted into the lumen 40 of the needle body 36. That is, an inner diameter of the needle body 36 is larger than an outer diameter of the light guide member 22.

The needle body 36 is made of a metal material such as, for example, stainless steel, aluminum, an aluminum alloy, titanium, and a titanium alloy. Note that, the needle body 36 may be made of a resin material such as polypropylene (PP), polycarbonate (PC), polyether ether ketone (PEEK), and liquid crystal polymer (LCP).

In a case where the needle body 36 has transparency, a light leakage prevention portion not illustrated is provided on an outer peripheral surface or an inner peripheral surface of the needle body 36. The light leakage prevention portion is formed of an opaque material. The light leakage prevention portion is formed by, for example, coating a surface of the needle body 36 with carbon black. The light leakage prevention portion may be formed by coating the surface of the needle body 36 with a metal mirror, a multilayer film filter or the like.

The needle body 36 is formed to be sufficiently longer than the catheter shaft 24. The needle body 36 protrudes in a distal direction from the distal end of the catheter shaft 24 in the initial state of the blood vessel puncture device 10.

As illustrated in Fig. 4A, a blade surface 42 inclined with respect to an axis of the needle body 36 is formed at the distal end of the needle body 36. A distal end opening 44 communicating with the lumen 40 of the needle body 36 is formed on the blade surface 42.

As illustrated in Fig. 3, the needle hub 38 includes a needle connection hole 46, a lumen 48, a first connection portion 50, a blood inflow portion 52, and a filter 54. The needle connection hole 46 is formed at a distal end of the needle hub 38. The proximal end of the needle body 36 is inserted into the needle connection hole 46. The proximal end of the needle body 36 is fixed to an inner peripheral surface of the needle hub 38 forming the needle connection hole 46. A position of the proximal end of the needle body 36 is located in the distal direction from a position of a proximal end of the needle connection hole 46.

The lumen 48 of the needle hub 38 communicates with the proximal end of the needle connection hole 46 and opens on a proximal end face of the needle hub 38. A guide surface 56 for guiding the light guide member 22 to the lumen 40 of the needle body 36 is provided on the inner peripheral surface of the needle hub 38 forming the lumen 48. The guide surface 56 is tapered in diameter toward the needle body 36 (in the distal direction). The first connection portion 50 is provided at a proximal end of the needle hub 38. The first connection portion 50 is a recess (hole) that opens on the proximal end face of the needle hub 38 and communicates with the lumen 48 of the needle hub 38.

The blood inflow portion 52 is a portion for confirming flashback. The blood inflow portion 52 includes a first opening 58 that opens on the inner peripheral surface forming the needle connection hole 46 of the needle hub 38 and a second opening 60 that opens on the proximal end face of the needle hub 38. A position of the first opening 58 is located in the proximal direction from the proximal end of the needle body 36. The second opening 60 is covered with the filter 54 fixed to the proximal end face of the needle hub 38. The filter 54 allows air to circulate and prevents blood from circulating. The filter 54 functions as a blood circulation prevention unit that prevents the blood flowing into the blood inflow portion 52 from circulating in the light source unit 20.

An area of the first opening 58 of the blood inflow portion 52 is larger than a cross-sectional area of an annular flow path formed between an outer peripheral surface of the light guide member 22 and an inner peripheral surface of the needle connection hole 46 in the proximal direction from the first opening 58 in the needle connection hole 46. Therefore, the blood derived from the lumen 40 of the needle body 36 to the needle connection hole 46 preferentially flows to the blood inflow portion 52. That is, it is possible to effectively suppress the blood from flowing to the lumen 48 of the needle hub 38.

Examples of a constituent material of the needle hub 38 are similar to the examples of the constituent material of the needle body 36 described above. A wall portion of the needle hub 38 includes a visual recognition portion 62 for visually recognizing the blood flowing into the blood inflow portion 52 from outside. The visual recognition portion 62 has transparency.

The light source unit 20 is detachably formed at the proximal end of the needle hub 38. When the blood vessel 302 is punctured with the needle body 36 in a state in which the light source unit 20 is attached to the needle hub 38, a surface of the light source unit 20 facing the living body site 300 is formed in a planar shape (having no protrusion). In this case, it is possible to suppress the light source unit 20 from hitting the living body site 300 when the needle body 36 punctures the blood vessel 302.

The light source unit 20 includes a light source unit 64, a power supply unit 66, and a housing 68. The light source unit 64 is accommodated in the housing 68. The light source unit 64 emits light L1 in the distal direction. As the light source unit 64, for example, an LED, a lamp (for example, a halogen lamp), a laser irradiation unit (for example, a laser diode) or the like is used. The light source unit 64 preferably emits the light L1 having directivity. Note that, the light source unit 64 may emit the light L1 having no directivity.

The light source unit 64 emits the light L1 in a wavelength region that is easily absorbed by hemoglobin in blood and easily transmitted through skin tissue 304. Specifically, the light source unit 64 emits at least one of visible light and near-infrared light. A wavelength of the visible light is preferably 600 nm or longer and shorter than 700 nm. A wavelength of the near-infrared light is 700 nm or longer and 2500 nm or shorter, preferably 700 nm or longer and 1400 nm or shorter, and more preferably 780 nm or longer and 940 nm or shorter.

The light L1 emitted by the light source unit 64 has a peak wavelength of 600 nm or longer and 2500 nm or shorter. The light source unit 64 may simultaneously emit the light L1 of a plurality of wavelengths. That is, the light source unit 64 may simultaneously emit the visible light of 600 nm and near-infrared light of 850 nm, for example. The light source unit 64 can adjust luminance of the light L1. The light source unit 64 may be continuously lit or blink.

The power supply unit 66 is provided at a proximal end of the housing 68. The power supply unit 66 supplies power to the light source unit 64. The power supply unit 66 includes, for example, a battery. Examples of the battery include a primary battery, a secondary battery, a solar battery and the like. The power supply unit 66 may include a wireless power supply device or may include an insertion plug connectable to a wiring plug connector (outlet). The power supply unit 66 may be detachably attached to the proximal end of the housing 68 or may be undetachably fixed. In a case where the power supply unit 66 is detachably attached to the housing 68, an attaching structure between the power supply unit 66 and the housing 68 may be an uneven fitting structure, a claw engagement structure, an adsorption structure using a magnet, an engagement structure using a hook-and-loop fastener, a screw engagement structure using a screw or the like. The power supply unit 66 may be accommodated in the housing 68.

The housing 68 is formed in a tubular shape (for example, a cylindrical shape). The housing 68 includes a housing main body 72 in which an accommodation space 70 of the light source unit 64 is formed, and a second connection portion 74 that protrudes in the distal direction from a distal end of the housing main body 72 and is detachably attached to the first connection portion 50 of the needle hub 38.

The second connection portion 74 is a protrusion that can be fitted to the first connection portion 50, which is the recess. That is, a connection structure between the first connection portion 50 and the second connection portion 74 is an uneven fitting structure. Note that, the connection structure between the first connection portion 50 and the second connection portion 74 may be a claw engagement structure, an attraction structure by a magnet, an engagement structure by a hook-and-loop fastener, a screw engagement structure by a screw or the like. At least one of the first connection portion 50 and the second connection portion 74 may have a positioning structure not illustrated for positioning the light guide member 22 in such a manner that a light emission unit 88 (refer Fig. 4A) to be described later of the light guide member 22 faces the distal end opening 44 of the needle body 36. The positioning structure is, for example, an uneven structure and the like.

The second connection portion 74 also serves as a support that supports a proximal end of the light guide member 22. The light guide member 22 is supported by the second connection portion 74 in such a manner that an axis of the light guide member 22 is coaxial with an optical axis of the light source unit 64. Note that, the light guide member 22 may be detachable from the light source unit 20.

A proximal end face of the light guide member 22 faces the light source unit 64. A space is provided between the proximal end face of the light guide member 22 and the light source unit 64. A condenser lens not illustrated that condenses the light L1 emitted by the light source unit 64 on the light guide member 22 may be arranged in a space between the proximal end face of the light guide member 22 and the light source unit 64. Examples of a constituent material of the condenser lens include glass, a resin material (for example, acrylic resin) and the like, for example.

Examples of a constituent material of the housing 68 include the constituent material of the needle body 36 described above, for example. The housing 68 is made of, for example, a material that does not transmit the light L1 emitted by the light source unit 64. The housing 68 may be made of a material having transparency. In this case, the light leakage prevention portion is provided on an outer peripheral surface or an inner peripheral surface of the housing 68. The light leakage prevention portion of the housing 68 is formed in a manner similar to that of the light leakage prevention portion of the needle body 36.

The outer peripheral surface of the housing 68 preferably has a smooth surface without unevenness so as to be easily subjected to alcohol wiping. In a state in which the second connection portion 74 is attached to the first connection portion 50, the light guide member 22 and the light source unit 64 are located on the axis of the needle body 36.

The light guide member 22 linearly extends in the axial direction of the needle body 36. When the light source unit 20 is attached to the needle hub 38, the light guide member 22 is inserted into the lumen 48 of the needle hub 38, the needle connection hole 46, and the lumen 40 of the needle body 36 from a site in the proximal direction from the needle hub 38. The axis of the light guide member 22 is located on the axis of the needle body 36. Note that, the position of the axis of the light guide member 22 may be offset with respect to the position of the axis of the needle body 36.

As illustrated in Fig. 4A, the outer diameter of the light guide member 22 is smaller than the inner diameter of the needle body 36. That is, a blood introduction path 76 for guiding the blood in the proximal direction is formed between the outer peripheral surface of the light guide member 22 and the inner peripheral surface of the needle body 36.

The light guide member 22 is formed to be solid. The light guide member 22 guides the light L1 emitted by the light source unit 64 to the distal end opening 44 of the needle body 36. Examples of the light guide member 22 include an optical fiber, an acrylic rod, a glass rod, a light emitting tub or the like. As illustrated in Figs. 4A and 4B, at least a part of the outer surface of the light guide member 22 is provided with a light non-transmissive portion 78 that prevents emission of the light L1 emitted by the light source unit 64. Note that, the proximal end face of the light guide member 22 is not covered with the light non-transmissive portion 78 but is exposed.

The light non-transmissive portion 78 is formed by, for example, coating a surface of the light guide member 22 with carbon black. The light non-transmissive portion 78 may be formed by coating the surface of the light guide member 22 with a metal mirror, a multilayer film filter or the like.

The light guide member 22 includes a light guide distal end 82 protruding in the distal direction from a proximal end 80 of the distal end opening 44 of the needle body 36. A distal end of the light guide distal end 82 is located in the proximal direction from a distal end 84 of the distal end opening 44 of the needle body 36. Specifically, the distal end of the light guide distal end 82 is located in the proximal direction from a center 86 of the distal end opening 44 of the needle body 36 in the axial direction of the needle body 36 (refer to Fig. 4B). The light guide distal end 82 does not protrude outward from the distal end opening 44 of the needle body 36. As a result, the living body site 300 can be smoothly punctured with the blade surface 42. Note that, at least a part of the light guide distal end 82 may protrude outward from the distal end opening 44.

The light emission unit 88 that is not covered with the light non-transmissive portion 78 is provided on an outer peripheral surface of the light guide distal end 82. The light emission unit 88 emits the light L1 in a direction in which the distal end opening 44 of the needle body 36 faces. The light non-transmissive portion 78 is provided on an entire outer peripheral surface of the light guide member 22 except for the light emission unit 88. The light emission unit 88 extends in the axial direction of the light guide member 22. The light emission unit 88 is formed in, for example, an elliptical shape.

As illustrated in Fig. 4B, a length of the light emission unit 88 in a circumferential direction of the light guide distal end 82 is equal to or shorter than a half circumference of the light guide distal end 82. Specifically, the length of the light emission unit 88 in the circumferential direction of the light guide distal end 82 is equal to or shorter than an eighth circumference of the light guide distal end 82. In this case, the light L1 emitted from the light emission unit 88 can be suppressed from spreading in the circumferential direction of the light guide distal end 82. A proximal end of the light emission unit 88 is located in the distal direction from the proximal end 80 of the distal end opening 44 of the needle body 36. The size, shape, and number of the light emission unit 88 can be designed appropriately.

The light non-transmissive portion 78 is provided on an entire distal end face 821 of the light guide distal end 82. As a result, the light L1 can be suppressed from being emitted in the distal direction of the light guide member 22. That is, the light L1 emitted in the distal direction of the light guide member 22 is suppressed from being irregularly reflected in the skin tissue 304, and a light emitting region of the light guide member 22 is suppressed from being excessively expanded.

The light guide member 22 is not limited to the configuration described above. The light guide member 22 may be formed in a hollow (tubular) shape, and a lumen of the light guide member 22 may function as the blood introduction path 76 for guiding the blood to the blood inflow portion 52. In this case, the light non-transmissive portion 78 is also provided on an inner peripheral surface of the light guide member 22. As a result, the light L1 can be suppressed from being emitted in the distal direction of the light guide member 22 via the lumen of the light guide member 22.

The light non-transmissive portion 78 is not required to be provided on the outer peripheral surface of a portion (light guide base 90) in the proximal direction from the light guide distal end 82 of the light guide member 22. In a case where the light non-transmissive portion 78 is not provided on the outer peripheral surface of the light guide base 90 and the needle hub 38 is made of a material having transparency, it is preferable that the light leakage prevention portion is provided on an outer peripheral surface or the inner peripheral surface of the needle hub 38. Note that, the light leakage prevention portion is not provided in the visual recognition portion 62 of the needle hub 38. The light leakage prevention portion of the needle hub 38 is formed in the manner similar to that of the light leakage prevention portion of the needle body 36.

As illustrated in Fig. 1, the visualization device 14 is provided with an irradiation unit 92, a light reception unit 94, and an image display unit 96. The irradiation unit 92 irradiates the living body site 300 (visualization target object) punctured with the blood vessel puncture device 10 with light L2. The irradiation unit 92 includes an irradiation unit main body 98 that emits the light L2. The irradiation unit main body 98 emits near-infrared light as the light L2. A wavelength of the near-infrared light emitted by the irradiation unit main body 98 is 700 nm or longer and 2500 nm or shorter, preferably 700 nm or longer and 1400 nm or shorter, and more preferably 780 nm or longer and 940 nm or shorter. Note that, the wavelength of the light L1 and the wavelength of the light L2 are preferably different from each other. The wavelength of the light L1 and the wavelength of the light L2 may be the same.

The light reception unit 94 is arranged on a side opposite to the irradiation unit 92 across the living body site 300. In other words, the irradiation unit 92 and the light reception unit 94 are arranged to face each other across the living body site 300. The light reception unit 94 is a camera (imaging unit) for receiving the light L1 emitted by the light source unit 64 and the light L2 emitted by the irradiation unit 92. For example, a CCD camera and the like for near-infrared light is used as the light reception unit 94. The light reception unit 94 may be a film capable of converting the near-infrared light into the visible light.

The image display unit 96 displays an image (received light image 100) created on the basis of the light L2 received by the light reception unit 94. The image display unit 96 may be goggles attachable and detachable by a person, a stationary display, or a projector.

In the visualization device 14, the irradiation unit 92 may be arranged on a side on which the light reception unit 94 is located with respect to the living body site 300. In this case, the light reception unit 94 receives the light L2 (reflected light) reflected by the living body site 300 out of the light L2 emitted by the irradiation unit 92. In the visualization device 14, the irradiation unit 92 may be arranged on both the side opposite to the light reception unit 94 across the living body site 300 and the side on which the light reception unit 94 is located with respect to the living body site 300. In this case, out of the light L2 emitted by each irradiation unit 92, both the light L2 transmitted through the living body site 300 (transmitted light) and the light L2 reflected by the living body site 300 (reflected light) are received by the light reception unit 94.

Next, an example of a puncturing procedure of the blood vessel 302 by the blood vessel puncture device 10 will be described.

In the initial state of the blood vessel puncture device 10, the blade surface 42 protrudes in the distal direction from the distal end opening 34 of the catheter shaft 24 in a state of facing upward (refer to Fig. 3). Note that, the light source unit 20 is not attached to the needle hub 38. Here, a case where the light source unit 64 emits the light L1 including the near-infrared light will be described.

First, a user determines necessity of the light source unit 20 on the basis of the blood vessel 302 to be punctured of a patient. Specifically, in a case of determining that the blood vessel 302 to be punctured is easily externally grasped and the light source unit 20 is unnecessary, the user performs a puncturing operation of the needle body 36 with respect to the blood vessel 302 without attaching the light source unit 20 to the needle hub 38.

In contrast, in a case of determining that it is difficult to externally grasp the blood vessel 302 to be punctured and the light source unit 20 is necessary, the user attaches the second connection portion 74 of the light source unit 20 to the first connection portion 50 of the needle hub 38.

Subsequently, the user sets the visualization device 14. Specifically, as illustrated in Fig. 5, the irradiation unit 92 is arranged below the living body site 300 (for example, the forearm of the human body) and the light reception unit 94 is arranged above the living body site 300. Then, the living body site 300 is irradiated with the light L2 by the irradiation unit 92, and the living body site 300 (a position on a skin side than the blood vessel 302) is punctured with the distal end of the needle body 36.

Then, the light L2 emitted by the irradiation unit 92 is transmitted through the skin tissue 304 of the living body site 300 while being scattered, and the transmitted light L2 (transmitted light) is received by the light reception unit 94. At that time, hemoglobin in the blood flowing in the blood vessel 302 absorbs the light L2. The light L2 does not transmit through the needle body 36.

The light L1 emitted by the light source unit 64 is guided in the distal direction via the light guide member 22 and emitted from the light emission unit 88 in a direction (upward) in which the distal end opening 44 of the needle body 36 faces. The light L1 emitted from the light emission unit 88 is transmitted through the skin tissue 304 of the living body site 300 while being scattered, and the transmitted light L1 (transmitted light) is received by the light reception unit 94. At that time, the light L1 is not emitted in the distal direction of the light guide member 22. Therefore, irregular reflection of the light L1 in the distal direction of the light guide member 22 is suppressed.

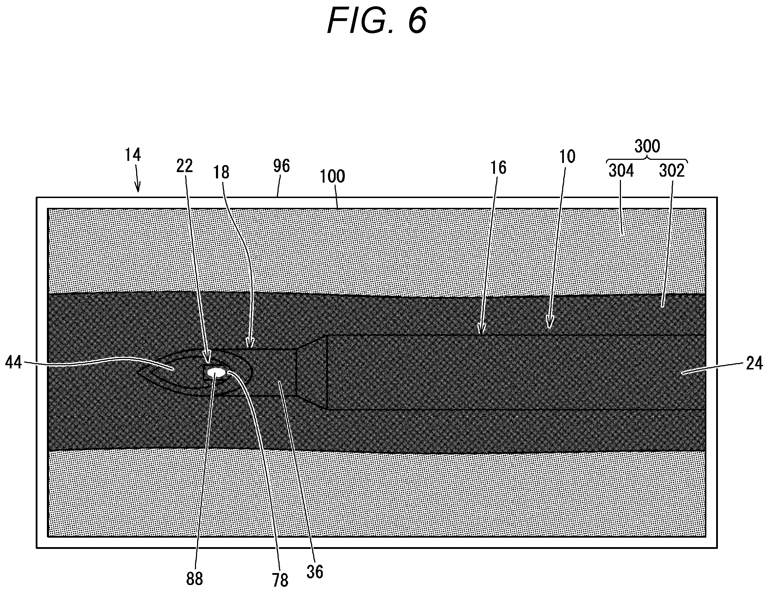

As a result, as illustrated in Fig. 6, the received light image 100 created on the basis of the light L2 received by the light reception unit 94 is displayed on the image display unit 96. In the received light image 100, for example, the skin tissue 304, the blood vessel 302, the distal end of the needle body 36 and the like are displayed.

Specifically, in the received light image 100, the light emission unit 88 of the light guide member 22 is displayed the brightest (in white), and the skin tissue 304 is displayed darker than the light emission unit 88. In the received light image 100, the blood vessel 302 and the needle body 36 are displayed darker than the skin tissue 304. As a result, the user can easily and clearly distinguish the blood vessel 302 and the needle body 36 in the received light image 100.

Subsequently, as illustrated in Fig. 7, when the distal end opening 44 of the needle body 36 is inserted into the blood vessel 302, the light L1 emitted by the light source unit 64 is absorbed by hemoglobin in the blood. Therefore, the light L1 does not reach the light reception unit 94, or even if the light L1 reaches the light reception unit 94, intensity thereof greatly decreases.

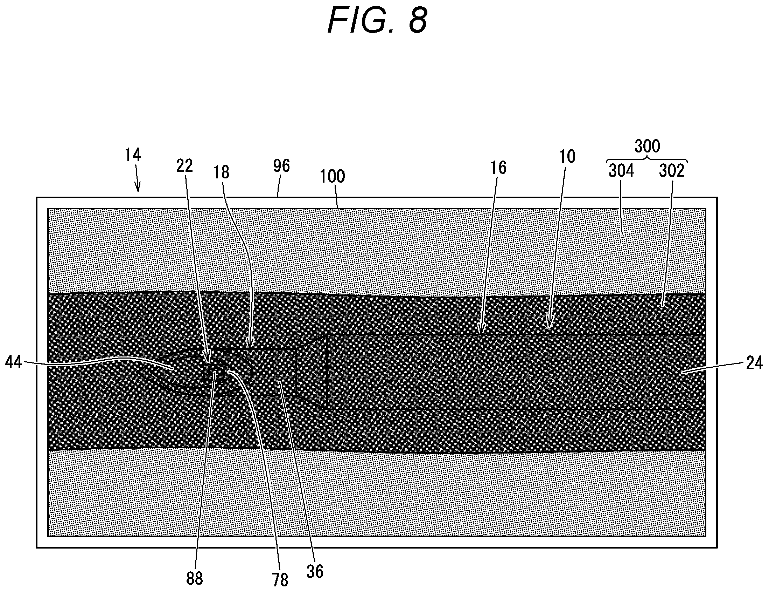

Therefore, as illustrated in Fig. 8, an appearance of the light guide distal end 82 changes (the light emission unit 88 becomes dark) in the received light image 100. In other words, luminance of the light emission unit 88 is equal to or less than luminance of the blood vessel 302. Note that, luminance of the needle body 36 is equal to or less than the luminance of the blood vessel 302. Therefore, the user can easily and quickly know that the distal end opening 44 of the needle body 36 is inserted into the blood vessel 302 (blood vessel securing of the needle body 36) from the received light image 100.

The blood in the blood vessel 302 is guided from the distal end opening 44 of the needle body 36 into the blood inflow portion 52 through the blood introduction path 76 of the needle body 36. At that time, air in the lumen 40 of the needle body 36 and the blood inflow portion 52 is discharged to the outside through the filter 54. Therefore, the blood is smoothly guided into the blood inflow portion 52 of the needle hub 38. Then, the user can know that the distal end opening 44 of the needle body 36 is inserted into the blood vessel 302 (blood vessel securing of the needle body 36) by visually recognizing the blood guided into the blood inflow portion 52.

At that time, a flow in the proximal direction of the blood in the blood inflow portion 52 is prevented by the filter 54. Therefore, the blood in the blood inflow portion 52 does not come into contact with the light source unit 20.

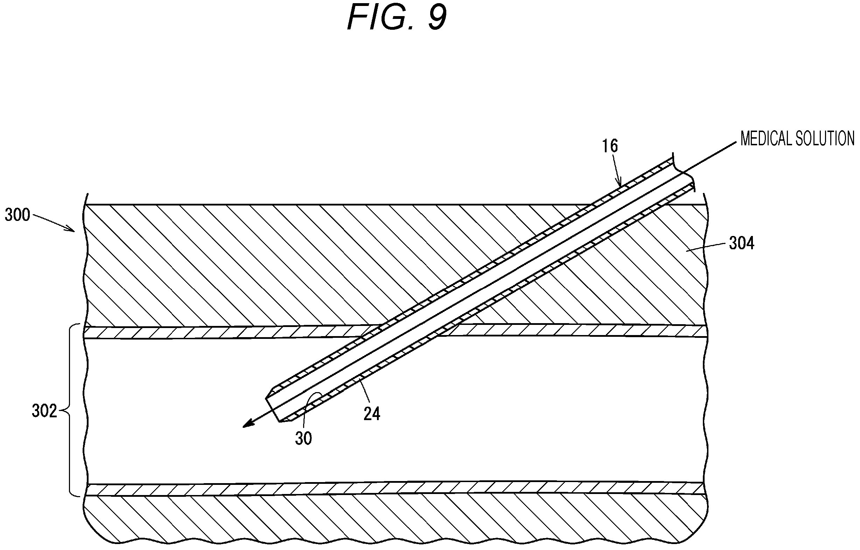

Thereafter, as illustrated in Fig. 9, the user removes the needle member 18 from the catheter member 16 in a state in which the catheter shaft 24 is indwelled in the blood vessel 302, and administers the medical solution into the blood vessel 302 via the lumen 30 of the catheter shaft 24.

The light source unit 20 is detached from the needle hub 38, and the light guide member 22 is detached from the light source unit 20. The light source unit 20 is reused. Note that, the used needle member 18 and light guide member 22 are discarded.

Note that, in the present embodiment, in a case where the light source unit 64 emits only the visible light, the user can recognize the light L1 emitted from the light emission unit 88 in a state before the blood vessel securing of the needle body 36. In contrast, since the light L1 is absorbed by hemoglobin in the blood in a state in which the blood vessel is secured by the needle body 36, the user cannot recognize the light L1 emitted from the light emission unit 88. Therefore, even in a case where the light source unit 64 emits only the visible light, it is possible to easily and quickly know that the blood vessel is secured by the needle body 36 on the basis of the light L1 emitted by the light source unit 64.

The present embodiment has the following effects.

According to the present embodiment, the light L1 emitted by the light source unit 20 is emitted from the light emission unit 88 of the light guide distal end 82 through the distal end opening 44 of the needle body 36. Therefore, it is possible to quickly know that the distal end opening 44 of the needle body 36 is inserted into the blood vessel 302. Since the outer surface of the light guide distal end 82 includes the light non-transmissive portion 78, an amount of the light L1 emitted from the light guide distal end 82 can be suppressed. As a result, irregular reflection in the skin tissue 304 is suppressed and the light emitting region of the light guide distal end 82 is narrowed, so that the position of the distal end opening 44 of the needle body 36 in the skin tissue 304 can be known more accurately. Therefore, the needle body 36 can accurately puncture the blood vessel 302.

The light non-transmissive portion 78 is provided on the distal end face 821 of the light guide distal end 82.

According to such a configuration, since the amount of light emitted in the distal direction of the needle body 36 can be suppressed, the light emitting region of the light guide distal end 82 in the skin tissue 304 can further be narrowed. Therefore, the needle body 36 can more accurately puncture the blood vessel 302.

The light non-transmissive portion 78 is provided on an entire distal end face 821 of the light guide distal end 82.

According to such a configuration, it is possible to further suppress the irregular reflection in the skin tissue 304.

The light non-transmissive portion 78 is provided on the outer peripheral surface of the light guide distal end 82.

According to such a configuration, since unnecessary emission of the light L1 from the outer peripheral surface of the light guide distal end 82 can be suppressed, the irregular reflection in the skin tissue 304 can further be suppressed.

A length of the light emission unit 88 in the circumferential direction of the light guide distal end 82 is equal to or shorter than a half circumference of the light guide distal end 82.

According to such a configuration, it is possible to efficiently suppress the irregular reflection in the skin tissue 304.

The light source unit 20 emits the near-infrared light.

According to such a configuration, since the near-infrared light is more easily transmitted through the skin tissue 304 and absorbed by hemoglobin than the visible light, it is possible to more easily know that the distal end opening 44 of the needle body 36 is inserted into the blood vessel 302.

The light source unit 20 is detachably attached to the needle hub 38.

According to such a configuration, it is possible to select whether or not to cause the light guide distal end 82 to emit light according to the position of the blood vessel 302 and the skill of the user. The light source unit 20 can be reused.

The light guide member 22 is provided in the light source unit 20. An inner surface of the needle hub 38 includes a guide surface 56 tapered in diameter toward the lumen 40 of the needle body 36.

According to such a configuration, when the light source unit 20 is attached to the needle hub 38, the light guide base 90 can be easily inserted into the lumen 40 of the needle body 36 by the guide surface 56.

The needle body 36 or the light guide member 22 is provided with the blood introduction path 76 for guiding the blood to the needle hub 38. The needle hub 38 is provided with the blood inflow portion 52 for confirming flashback into which the blood guided from the blood introduction path 76 flows.

According to such a configuration, it is possible to reconfirm that the distal end opening 44 of the needle body 36 is inserted into the blood vessel 302 by the inflow of the blood into the blood inflow portion 52.

The filter 54 (blood circulation prevention unit) that prevents the blood flowing into the blood inflow portion 52 from circulating in the light source unit 20 is provided in the needle hub 38.

According to such a configuration, the filter 54 can prevent the light source unit 20 from coming into contact with the blood. Therefore, in a case where the light source unit 20 is detachably attached to the needle hub 38, the light source unit 20 can be reused.

The blood vessel puncture system 12 includes the blood vessel puncture device 10, the light reception unit 94 that receives the light L1 derived from the light emission unit 88, and the image display unit 96 that displays the received light image 100 created on the basis of the light L1 received by the light reception unit 94.

According to such a configuration, the position of the distal end opening 44 of the needle body 36 can be easily confirmed even in a case where the light L1 emitted from the light emission unit 88 is difficult to be visually recognized or cannot be visually recognized with the naked eye.

### (First Modification)

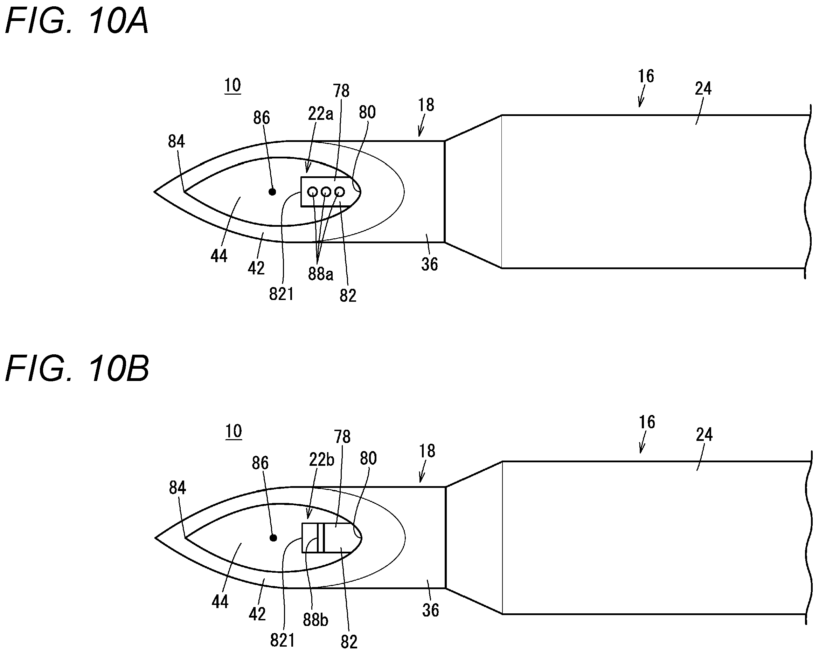

Next, a light guide member 22a according to a first modification will be described. As illustrated in Fig. 10A, the light guide member 22a is provided with a plurality of light emission units 88a. The light emission unit 88a is formed in, for example, a perfect circular shape. The plurality of light emission units 88a is arranged at intervals in an axial direction of the light guide member 22a. In an example in Fig. 10A, the plurality of light emission units 88a is arranged at regular intervals in the axial direction of the light guide member 22a.

In the present modification, the plurality of light emission units 88a is arranged at intervals in the axial direction of the light guide member 22a.

According to such a configuration, when the distal end opening 44 of the needle body 36 is inserted into the blood vessel 302, the light emission unit 88a becomes invisible stepwise from a distal direction, so that a user can easily grasp to what extent the distal end opening 44 of the needle body 36 is inserted into the blood vessel 302.

### (Second Modification)

Next, a light guide member 22b according to a second modification will be described. As illustrated in Fig. 10B, the light guide member 22b is provided with a light emission unit 88b annularly extending in a circumferential direction of a light guide distal end 82. A length of the light emission unit 88b in an axial direction of the light guide member 22b can be appropriately set.

In the present modification, the light emission unit 88b annularly extends in a circumferential direction of the light guide distal end 82.

According to such a configuration, it is not necessary to position the light emission unit 88b and a distal end opening 44 of a needle body 36 in the circumferential direction of the light guide distal end 82.

### (Configuration Example)

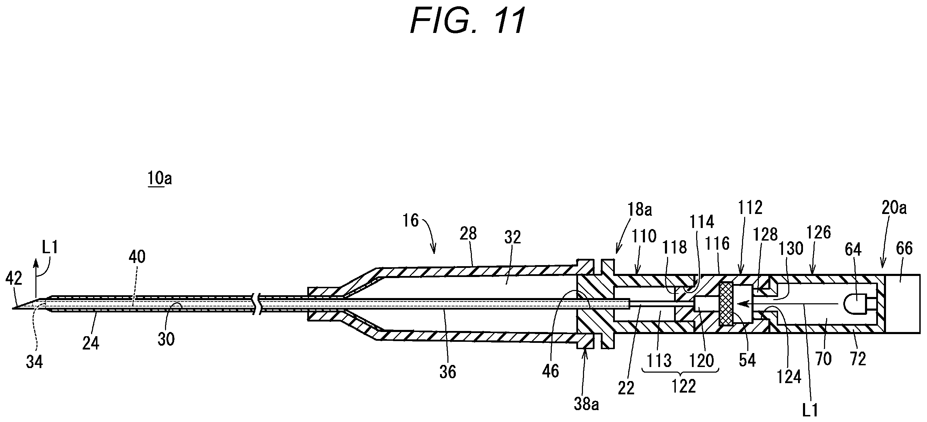

Next, a blood vessel puncture device 10a according to a configuration example is described. In the present configuration example, the same components as those of the above-described blood vessel puncture device 10 are denoted by the same reference numerals, and the detailed description thereof will be omitted. As illustrated in Fig. 11, the blood vessel puncture device 10a includes a catheter member 16, a needle member 18a, a light source unit 20a, and a hollow (tubular) light guide member 22.

The needle member 18a includes a needle body 36 and a needle hub 38a. The needle hub 38a includes a first member 110 and a second member 112. The first member 110 includes a needle connection hole 46, a lumen 113, and a first engagement portion 114. A proximal end of the needle body 36 is located at a proximal end of the needle connection hole 46. Note that, the proximal end of the needle body 36 can be located in a distal direction from the proximal end of the needle connection hole 46. The first engagement portion 114 is a recess (hole) that opens on a proximal end face of the first member 110 and communicates with the lumen 113 of the first member 110.

The second member 112 includes a support 116 and a filter 54. The support 116 is formed in a hollow shape. The support 116 supports the filter 54 arranged in the support 116. A second engagement portion 118 that engages with the first engagement portion 114 is provided at a distal end of the support 116. The second engagement portion 118 is a protrusion that fits to the first engagement portion 114, which is the recess. Note that, the first engagement portion 114 may be a protrusion, and the second engagement portion 118 may be the recess. The support 116 is fixed to the first member 110. The support 116 may be integrally molded with the first member 110.

The second engagement portion 118 supports a proximal end of the light guide member 22. In other words, the proximal end of the light guide member 22 is fixed to the second engagement portion 118. The support 116 includes a blood flow path 120 in a proximal direction of the second engagement portion 118. The blood flow path 120 communicates with a lumen of the light guide member 22. The lumen 113 of the first member 110 and the blood flow path 120 form a blood inflow portion 122 for confirming flashback through which blood flowing from a distal end opening 44 of the needle body 36 is guided. Note that, the filter 54 is arranged so as to close the blood flow path 120 from the proximal direction.

An attachment portion 124 for attaching the light source unit 20a is provided in the proximal direction from the filter 54 of the support 116. The attachment portion 124 is a recess (hole) that opens on a proximal end face of the support 116. The attachment portion 124 is located on an axis of the needle body 36.

The light source unit 20a is detachably formed in the attachment portion 124. The light source unit 20a includes a light source unit 64, a power supply unit 66, and a housing 126. The housing 126 includes a housing main body 72 and a connection portion 128. The connection portion 128 is a protrusion that can be fitted to the attachment portion 124, which is the recess. A light derivation hole 130 communicating with an accommodation space 70 is formed in the connection portion 128. The light derivation hole 130 opens on a distal end face of the connection portion 128.

In the present configuration example, it is not necessary to remove the light guide member 22 when detaching the light source unit 20a from the needle hub 38a. In other words, after the blood vessel puncture device 10a is used, the light guide member 22 can be discarded together with the needle hub 38a as a disposable product. Since the light source unit 20a is detachably attached to the second member 112, blood exposure of the light source unit 20a can be efficiently reduced.

In the present configuration example, the blood vessel puncture device 10a may include the light guide member 22a or the light guide member 22b described above instead of the light guide member 22.

The blood vessel puncture device 10 is not limited to the configuration described above. At least a part of the light guide member 22 may be fixed to the needle member 18. The light guide member 22 may be formed separately into a first portion fixed to the needle member 18 and a second portion fixed to the light source unit 20. In this case, when the light source unit 20 is attached to the needle hub 38, the first portion and the second portion are optically connected to form a continuous optical path. An entire light guide member 22 may be fixed to the needle member 18. In this case, the light source unit 20 can be easily attached to and detached from the needle hub 38. The light guide member 22 may be discontinuous in the axial direction of the light guide member 22. The light source unit 20 may be fixed to the needle hub 38 so as not to be detachable.

The blood vessel puncture device 10 is not limited to the configuration described above. The light emission unit 88 may be provided on the distal end face 821 of the light guide distal end 82.

Note that, the present invention is not limited to the above disclosure, and various configurations can be adopted without departing from the gist of the present invention.

### Reference Signs List

- 10, 10a: Blood vessel puncture device
- 12: Blood vessel puncture system
- 20: Light source unit
- 22, 22a, 22b: Light guide member
- 24: Catheter shaft
- 28: Catheter hub
- 36: Needle body
- 38, 38a: Needle hub
- 40: Lumen of needle body
- 42: Blade surface
- 44: Distal end opening of needle body
- 52: Blood inflow portion
- 54: Filter (blood circulation prevention unit)
- 56: Guide surface
- 76: Blood introduction path
- 78: Light non-transmissive portion
- 80: Proximal end of distal end opening
- 82: Light guide distal end
- 88, 88a, 88b: Light emission unit
- 94: Light reception unit
- 96: Image display unit
- 100: Received light image
- 300: Living body site
- 302: Blood vessel
- L1, L2: Light

## Claims

1. A blood vessel puncture device comprising:
a tubular needle body capable of puncturing a blood vessel of a living body site;
a needle hub provided at a proximal end of the needle body; and
a light source unit provided in the needle hub,
a distal end of the needle body including:
a blade surface; and
a distal end opening formed on the blade surface and communicating with a lumen of the needle body, wherein
a light guide member for guiding light emitted by the light source unit to the distal end opening is arranged in the lumen of the needle body,
the light guide member includes:
a light guide distal end protruding in a distal direction from a proximal end of the distal end opening, and
an outer surface of the light guide distal end includes:
a light emission unit that emits the light in a direction in which the distal opening faces; and
a light non-transmissive portion that prevents emission of the light.

2. The blood vessel puncture device according to claim 1, wherein
the light non-transmissive portion is provided on a distal end face of the light guide distal end.

3. The blood vessel puncture device according to claim 2, wherein
the light non-transmissive portion is provided on an entire distal end face of the light guide distal end.

4. The blood vessel puncture device according to claim 1, wherein
the light emission unit and the light non-transmissive portion are provided on an outer peripheral surface of the light guide distal end.

5. The blood vessel puncture device according to claim 4, wherein
the light emission unit extends in an axial direction of the light guide member.

6. The blood vessel puncture device according to claim 4, wherein
a plurality of light emission units is arranged at intervals in an axial direction of the light guide member.

7. The blood vessel puncture device according to claim 4, wherein
the light emission unit extends annularly in a circumferential direction of the light guide distal end.

8. The blood vessel puncture device according to claim 4, wherein
a length of the light emission unit in a circumferential direction of the light guide distal end is equal to or less than a half circumference of the light guide distal end.

9. The blood vessel puncture device according to claim 1, wherein
the light source unit emits near infrared light.

10. The blood vessel puncture device according to claim 1, wherein
the light source unit is detachably attached to the needle hub.

11. The blood vessel puncture device according to claim 10, wherein
the light guide member is provided in the light source unit, and
an inner surface of the needle hub includes a guide surface tapered in diameter toward the lumen of the needle body.

12. The blood vessel puncture device according to claim 1, wherein
the needle body or the light guide member is provided with a blood introduction path for guiding blood to the needle hub, and
the needle hub is provided with a blood inflow portion for confirming flashback into which the blood guided from the blood introduction path flows.

13. The blood vessel puncture device according to claim 12, wherein
the needle hub is provided with a blood circulation prevention unit that prevents the blood flowing into the blood inflow portion from circulating in the light source unit.

14. The blood vessel puncture device according to claim 1, comprising:
a tubular catheter shaft including a lumen through which the needle body is inserted; and
a catheter hub provided at a proximal end of the catheter shaft and including a lumen through which the needle body is inserted.

15. A blood vessel puncture system comprising:
the blood vessel puncture device according to any one of claims 1 to 14;
a light reception unit that receives the light derived from the light emission unit; and
an image display unit that displays a received light image created on the basis of the light received by the light reception unit.
